# EUROPÄISCHE PATENTANMELDUNG

(11) **EP 0 958 828 A1**
(43) Veröffentlichungstag der Anmeldung: **24.11.1999**
(21) Anmeldenummer: 98810481.6
(22) Anmeldetag: 22.05.1998
(51) Int. Cl.: A61K 35/78

(54) **Präparat auf der Basis der Artischockenpflanze, insbesondere zur Verwendung als Arzneimittel oder Nahrungsergänzungsmittel**

(71) Anmelder: Greither, Peter, CH-9533 Kirchberg (CH)
(72) Erfinder: Engel, Dieter Wolfgang, 9524 Zuzwil (CH)
(74) Vertreter: Wenger, René

(57) **Zusammenfassung**

Die vorliegende Erfindung beschreibt ein Präparat aus einer Kombination aus mindestens einem Öl, welches mindestens eine ungesättigte ω-3-Fettsäure, bzw. mindestens ein Seetieröl enthält, und aus Bestandteilen der Artischockenpflanze. Das Präparat mit der erfindungsgemässen Kombination von Inhaltstoffen findet entweder Einsatz als Medikament oder aber als Nahrungsmittelergänzung.
Es richtet sich in erster Linie auf die Behandlung und Prävention von Hyperlipidämie, bzw. arteriosklerotischer Herz/Kreislauferkrankungen. In einer besonderen Ausführungsform werden dem erfindungsgemässen Präparat noch Bestandteilen der Knoblauchpflanze zugesetzt.

## Beschreibung

Die Erfindung betrifft ein Präparat, das eine Kombination aus mindestens einem Öl, welches mindestens eine Omega-3-mehrfach ungesättigte Fettsäure enthält, bzw. mindestens ein Seetieröl enthält, und Bestandteile der Artischockenpflanze.

Seit längerer Zeit ist aufgrund biochemischer Experimente und klinischer Untersuchungen bekannt, dass ω-3-mehrfach ungesättigte Fettsäuren (α-Linolensäure-Familie) eine prophylaktische und therapeutische Wirkung hinsichtlich arteriosklerotischer Herz-Kreislauferkrankungen aufweisen, wie z.B. publiziert in P.C. Weber, C.v. Schacky, R. Lorenz, "Deutscher Apothekerzeitung", 1986, *126*, S. 49 - 53. Der Einbau mehrfach ungesättigter Fettsäuren des ω-3-Typs in Plasmalipide und Zellmembranen der Menschen verbessert die Fliesseigenschaften des Blutes und reduziert die Klebrigkeit (Aggregabilität) der roten Blutkörperchen (Thrombozyten). Durch die herabgesetzte Trombozytenaggregation wird das Risiko von Arterienablagerungen und damit die Synthese von Low-Density-Cholesterol (LDL) vermindert. Die zusätzliche Wirkung, die sich in einer Senkung des Triglycerid-Spiegels und einer Hemmung einzelner Gerinnungsfaktoren (Fibrinogen) äussert, führt darüberhinaus zu einer Senkung des Blutdruckes und zu einer Verlängerung der Blutungszeit.

Mehrfach ungesättigte Fettsäuren des ω-3-Typs werden somit sowohl zur Prävention bzw. Behandlung arteriosklerotischer Herz-Kreislauf-Erkrankungen als auch für eine Senkung des LDL- und Triglycerid-Spiegels eingesetzt.

Omega-3-mehrfach ungesättigte Fettsäuren besitzen am dritten Kohlenstoffatom, gerechnet vom Methylende des Moleküls, eine Cis-Doppelbindung.

Als besonders wirksam haben sich speziell die ω-3-ungesättigte Fettsäuren Eikosapentaensäure EPA (C20:5ω-3) und Dokosahexaensäure (DHA) (C22:6ω-3) wie sie in Formel 1 und 2 dargestellt sind, erwiesen. 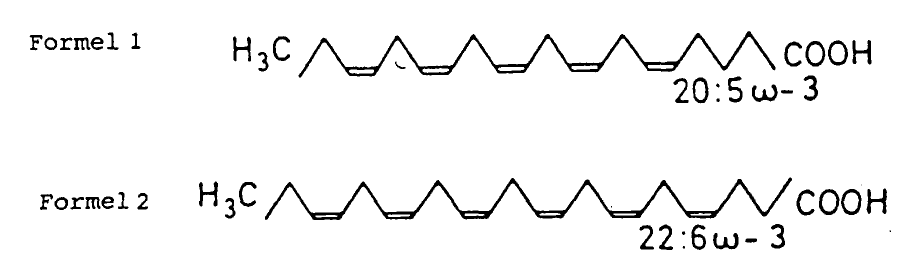 Diese beiden Säuren finden sich nach Anreicherung in der Nahrungskette in hohen Konzentrationen vor allem in Seetierölen, in Seetang und Phytoplankton (W.E.M. Lands, "Fish and Human Health", Academic Press, Orlando, 1986, S. 143 ff.). Omega-3-mehrfach ungesättigte Fettsäuren kommen daneben aber auch im Fleisch freilebender Wildtiere vor. Weiterhin können sie auch auf synthetischem Weg hergestellt werden.

Der Begriff Seetieröl wird als Sammelbezeichnung für Öle (Fette) aus Warmblütern (Wate, Robben) und aus Fischen sowie aus Fischteilen verstanden (Dr. Oetker, "Lexikon Lebensmittel und Ernährung", Ed. Firma Dr. August Oetker, 2. Auflage, 1983, Cerres-Verlag, Rudolf August Oetker KG, Bielefeld, Deutschland, Seite 518). Es wird unterschieden zwischen
1.) Fischölen (Fischfetten),
2.) Leberölen (Lebertranen) und
3.) Walölen (Waltranen) aus Meeressäugetieren.

Insbesondere Fischöle, die gewonnen werden aus dem Fleisch der Kaltwasserfische wie Makrele, Lachs, Hering, Anchovis und Thunfisch weisen spezifisch hohe Konzentrationen an EPA und DHA auf.

Es ist ebenfalls seit längerem bekannt, dass Bestandteile aus der Artischockenpflanze (Cynara scolymus) ebenfalls eine lipidsenkende und cholagoge Wirkung, trotz völlig anderer Wirkstoffe aufweisen. Unter dem Begriff "Artischockenpflanze" sollen definitionsgemäss alle Teile der Pflanze, d.h. ihre Wurzeln, Grünteile und Früchte fallen.

Überraschenderweise zeigte ein Präparat, welches eine Kombination aus
1. mindestens einem Öl, welches mindestens eine mehrfach ungesättigte ω-3-Fettsäure enthält bzw. mindestens ein Seetieröl, und
2. Bestandteile der Artischockenpflanze, (Cynara scolymus)
einen hinsichtlich ihrer Wirkeigenschaften sich verstärkenden Effekt. Die ω-3-mehrfach ungesättigten Fettsäuren können sowohl als freie Säuren, als Ester (z.B. Methyl-, Ethyl- oder Succinylester oder auch in Form ihrer Triglyceride eingesetzt werden. In der Regel liegen die ω-3-mehrfach ungesättigten Fettsäuren in einer Menge um die 12-70 % bezogen auf das Gewicht des Öles vor. Diese beschriebene Kombination aus ω-3-Fettsäure enthaltendem Öl und Artischockenpflanzenbestand-teilen wird als Medikament oder Nahrungsmittelergänzung eingesetzt.

Der synergistische Effekt dieses Präparates zeigt sich insbesondere bei der Behandlung bzw. Prävention von Hyperlipidämie, für die auch der Ausdruck Hyperlipoproteinämie gebräuchlich ist. Hyperlipidämie ist eine Störung des Lipidtransportes durch entweder erhöhte Synthese oder verzögerten Abbau von Lipoproteinen. Nach Art der vermehrten Lipide werden Hypertriglyceridämie, Hypercholesterinämie aber auch kombinierte Hyperlipidämien unterschieden (Römpp, "Chemielexikon", 9. Auflage, Ed. J. Falbe, M. Regiz, 1990, Seite 1916). Das erfindungsgemässe Präparat wirkt bevorzugt einem erhöhten Cholesterolspiegel entgegen, der wie bereits ausgeführt im Rahmen der Entstehung von Arteriosklerose eine bedeutende Rolle spielt. In erster Linie, aber nicht ausschliesslich, wird das erfindungsgemässe Präparat deshalb zur Behandlung und Prävention von Hyperlipidämie, insbesondere Hypercholesterinämie eingesetzt.

Als für das erfindungsgemässe Präparat geeignete Öle haben sich Fischöle, und insbesondere Fischöle aus dem Fischfleisch von Kaltwasserfischen, in einer Menge von 20 bis 80 Gew.% bezogen auf das Gewicht der Darreichungsform des Präparates erwiesen. Besonders vorteilhaft hinsichtlich der synergistischen Wirkung des Präparates haben sich Fischöle gezeigt, die Dokosahexaensäure (DHA) in 5 bis 80 Gew.% und Eikosapentaensäure (EPA) in einer Menge von 7 bis 60 Gew.% (bezogen auf das Gewicht des Fischöles) enthalten. Die Fischöle können erfindungsgemäss auch vor ihrem Einsatz mikroverkapselt werden und damit in fester, rieselfähiger Form vorliegen. Die Mikroverkapselung geschieht nach bekannten verfahren wie beschrieben in L. Lachmann, H.A. Lieberman, J.L. Kanig, "The Theory and Practice of Industrial Pharmacy", Lea & Febiger, 3. Auflage, Philadelphia, 1986, Kap 3, S. 412 - 429.

Die Bestandteile der Artischockenpflanze werden in Form aller gängigen Zubereitungen eingesetzt, wie z. B. beschrieben in P.H. List, D.P.C. Schmidt, "Technologie Pflanzlicher Arzneizubereitungen", Wissenschaftliche Verlagsgesellschaft, Stuttgart, Deutschland, 1984. Darunter fallen alle Extrakte, die in Form flüssiger oder pastöser hydrophiler oder hydrophober Lösungen oder Dispersionen aus der Artischockenpflanze gewonnen werden. Diese werden entweder direkt in flüssiger oder pastöser Form oder aber nach vorheriger Trocknung gemäss ebenfalls bekannter Verfahren in dem Präparat eingesetzt. Eine bevorzugte Einsatzform sind feste, pulverförmige Extrakte aus Artischockenblättern und/oder Artischockenfrüchten in einer Menge von 15 bis 60 Gew.% bezogen auf das Gesamtgewicht der jeweiligen Darreichungsform des Präparates. Werden die jeweiligen Artischockenpflanzenextrakte als hydrophile oder hydrophobe Lösungen oder Dispersionen direkt im Präparat eingesetzt, so beträgt ihre Menge 15 bis 80 Gew.% bezogen auf das Gesamtgewicht der jeweiligen Darreichungsform des Präparates.

Weiterhin zeigen getrocknete Artischockenblätter, die nach ihrer Trocknung pulverisiert werden, die gleichen vorteilhaften Wirkungen in einer Einsatzmenge von 15-80 % bezogen auf die jeweilige Darreichungsform.

In einer besonders bevorzugten und vorteilhaften Form werden die Bestandteile der Artischockenpflanze als Artischockenblätter- oder Artischockenfrüchte-Frischpflanzensaft in einer Menge von 30 bis 60 Gew.% bezogen auf das Gesamtgewicht der jeweiligen Darreichungsform des Präparates eingesetzt.

Frischpflanzensäfte haben den grossen Vorteil, dass die Wirkstoffe der Pflanze im Gegensatz zu den gängigen Extraktionsverfahren nicht verlorengehen oder verändert werden. In der Regel finden beim Extrahieren und Trocknen der Extrakte irreversible Fermentationen, Hydrolysevorgänge, Kondensationen, Oxidationen oder Verluste an flüchtigen Bestandteilen sowie andere Veränderungen der Wirkstoffe statt. Der Artischocken-Frischpflanzensaft wird durch eine zeitlich genau aufeinander abgestimmte Abfolge von Verfahrensschritten hergestellt. Einer Grobzerkleinerung und Pürierung bei Temperaturen bis 20°C und - wenn erforderlich - unter Stickstoff-Schutzatmosphäre folgt eine Filtrierung der Masse unter vollständigem Ausschluss von Lösungsmittel, wobei Wasser in dieser Definition eingeschlossen ist. Anschliessend wird der Rohsaft sauer eingestellt, uperisiert und nochmals filtriert. Der so erhaltene Frischpflanzensaft kann direkt eingesetzt werden oder aber nach vorheriger Trocknung. Die Trocknung sollte dann vorteilhaft innerhalb von 24 Stunden nach der Gewinnung des Frischpflanzensaftes bzw. spätestens innerhalb von 48 Stunden nach der Pressung bei maximalen Temperaturen bis 50°C durchgeführt werden.

In einer besonders vorteilhaften Ausführungsform werden dem erfindungsgemässen Präparat zusätzlich Bestandteile aus der Knoblauchpflanze (Allium sativum) in fester, pulverförmiger und/oder flüssiger Form in einer Menge von 5 bis 60 Gew.% bezogen auf die Darreichungsform des jeweiligen Präparates zugesetzt.

Es ist seit langem bekannt, dass die Inhaltsstoffe des Knoblauchpflanze ebenfalls anti-ateriosklerotische und die Blutfettwerte senkende Wirkungen haben. Daneben spielen sie für den Gefässschutz wegen ihrer anti-oxidativen Eigenschaften eine bedeutende Rolle (Römpp, "Chemielexikon", 9. Auflage, Ed. J. Falbe, M. Regiz, 1990, Seite 2264). Mit dem Begriff Knoblauchpflanze sollen auch in diesem Fall alle Pflanzen- und Früchtebestandteile erfasst sein. Die Präparation der Knoblauchpflanzenbestandteile erfolgt ebenfalls gemäss den in der Literatur beschriebenen Verfahren bzw. analog der Herstellung des Artischocken-Frischpflanzensaftes. In einer bevorzugten Anwendungsform werden die Knoblauchinhaltsstoffe mittels Mazeration gewonnen. Unter Mazeration wird das Extrahieren einer Droge mit einem Lösungsmittel unter täglich mehrmaligem Umschütteln oder Umrühren bei Raumtemperatur verstanden. Bevorzugt werden die Bestandteile aus der Knoblauchpflanze als Ölmazerate dem Präparat zugesetzt.

Die festen oder flüssigen Extrakte der Knoblauchpflanze bzw. die getrockneten und pulverisierten Knoblauchpflanzenbestandteile werden in einer Menge von 5-60 Gew.% bezogen auf das Gesamtgewicht der jeweiligen Darreichungsform eingesetzt.

Eine Kombination bestimmter Knoblauchextrakte und Fischöle ist bereits in EP 232 501 beschrieben.

Ebenfalls Teil der Erfindung ist ein Präparat, welches Seetieröl und Bestandteile der Artischockenpflanze enthält, zur Verwendung als Arzneimittel, insbesondere zur Behandlung und/oder Prophylaxe arteriosklerotischer Herz-Kreislauf-Erkrankungen und/oder zur Behandlung der Hypercholesterinämie.

Gegebenenfalls können auch diesem Präparat Bestandteile der Knoblauchpflanze zugegeben werden.

Das erfindungsgemässe Präparat wird gegebenenfalls zusammen mit üblichen weiteren Hilfestoffen wie z.B. PEG 400 oder Planzenöle wie beschrieben in L.Lachmann, H.A. Lieberman, J.L.Kanig, "The Theory and Practice of Industrial Pharmacy", 3. Auflage, Lea & Febiger, Philadelphia 1986, S. 398 ff. als feste oder halbfeste Darreichungsform bevorzugt in Weich- oder Hartgelatine bzw. Stärkekapseln eingeschlossen. Die Verkapselung ist in erster Linie aufgrund des unangenehmen Geruches der Öle, wenn als Fischöle oder Trane eingesetzt, notwendig. Die Kombination aus Ölen, Bestandteilen der Artischockenpflanze und gegebenenfalls der Knoblauchpflanze können in Form ihrer Mischungen verkapselt werden oder aber innerhalb der Kapsel in voneinander separierten Bereichen und damit unvermischt vorliegen. Letzere Variante ist vor allem in denjenigen Fällen notwendig, bei denen es zu störenden chemischen Wechselwirkungen zwischen den einzelnen Bestandteilen kommt. Neben der Verkapselung ist die Verarbeitung des erfindungsgemässen Präparates zu weiteren Darreichungsformen wie sie z.B. in Form von Dragées, Tabletten oder eingeschlossen in Ampullen vorliegen können, durchführbar.

Bei Darreichungsform als Kapsel, Tablette, etc. wird die äussere Hülle zur Angabe des Gesamtgewichtes dazugerechnet, da dort die äussere Hülle miteingenommen wird, bei Ampullen dagegen nicht.

Wenn erforderlich, werden die Kapseln, Tabletten oder Dragées durch Überzüge oder Vernetzungsreaktionen magensaftresistent umhüllt.

Die Erfindung wird nachfolgend anhand von Ausführungsbeispielen illustriert, ist jedoch nicht darauf limitiert.

### Beispiel 1:

200 mg Artischockenblätter-Extrakt in fester pulverförmiger Form, 400 mg Fischöl, welches mindestens 18 Gew.% Eikosapentaensäure und mindestens 12 Gew.% Dokosapentaensäure bezogen auf das Gewicht des Fischöls in Form ihrer Triglyceride enthält und 200 mg Knoblauchpulver werden mit 200 mg Hilfsstoffen versetzt und in eine 20 minims oblong Weichgelatinekapsel verkapselt.

Die Kapsel wird ggf. entweder durch Überzug mit HPMCP, CAP oder Eudragit S magensaftresistent gesprüht.

### Beispiel 2

500 mg getrockneter Artischockenfrüchte-Frischpflanzensaft wird mit 500 mg Fischöl, welches mindestens 30 Gew.% EPA und mindestens 20 Gew.% DHA bezogen auf das Gewicht des Fischöls in Form ihrer Triglyceride mit 100 mg Hilfsstoffen in eine 20 oblong Weichgelatinekapsel verkapselt.

### Beispiel 3

100 mg Artischockenblätterextrakt, 100 mg Knoblauchpulver und 200 mg mikroverkapseltes Fischöl werden mit 80 mg Hilfsstoffen versetzt und in eine Hartgelatinekapsel Grösse 0 abgefüllt.

### Beispiel 4

300 mg Artischockenblätterextrakt, 300 mg Knoblauchpulver und 200 mg sprühgetrocknetes Fischöl werden mit Hilfsstoffen zu einer Tablette verpresst. Diese Tablette wird wenn erforderlich durch Coaten mit Eutraget S magensaftresistent überzogen.

Beispiele 5 bis 25 werden in Tabelle 1 gezeigt.

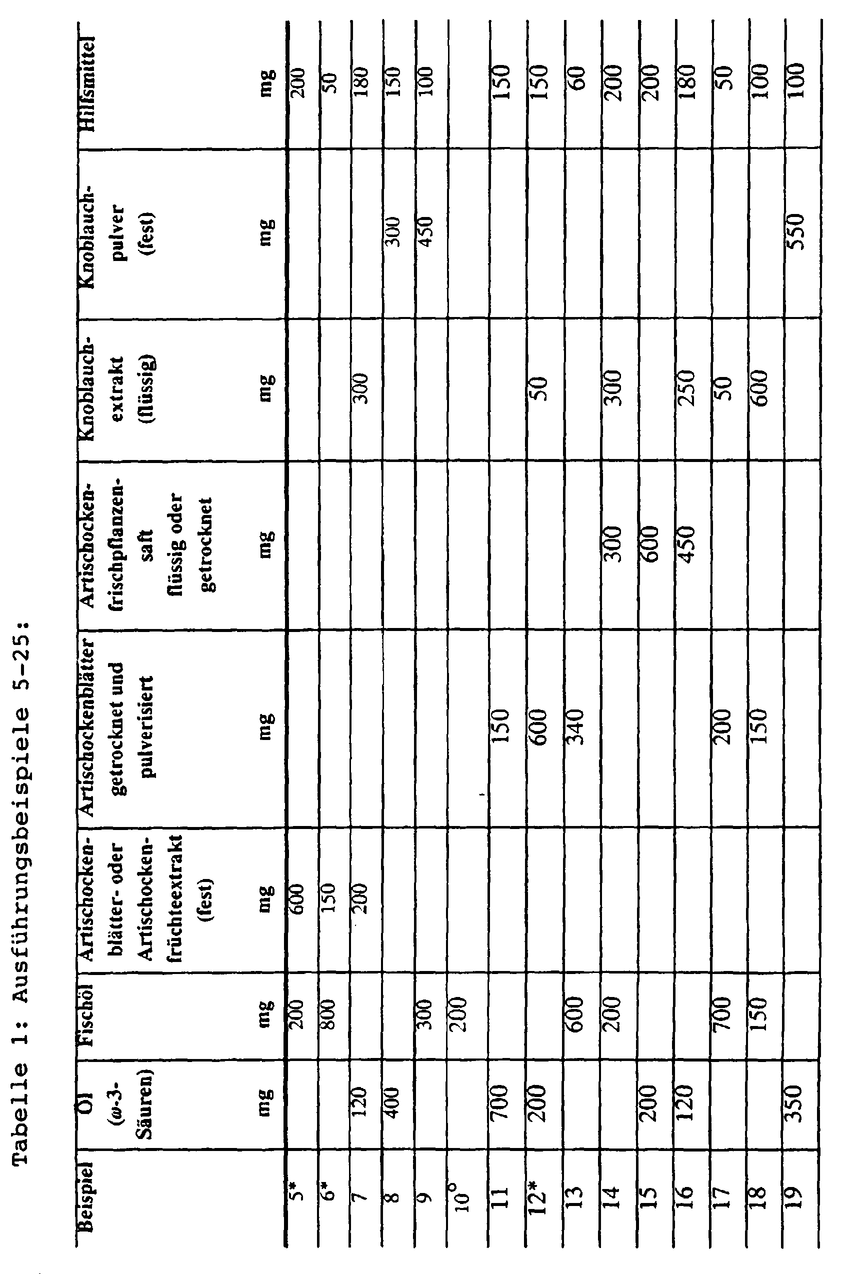

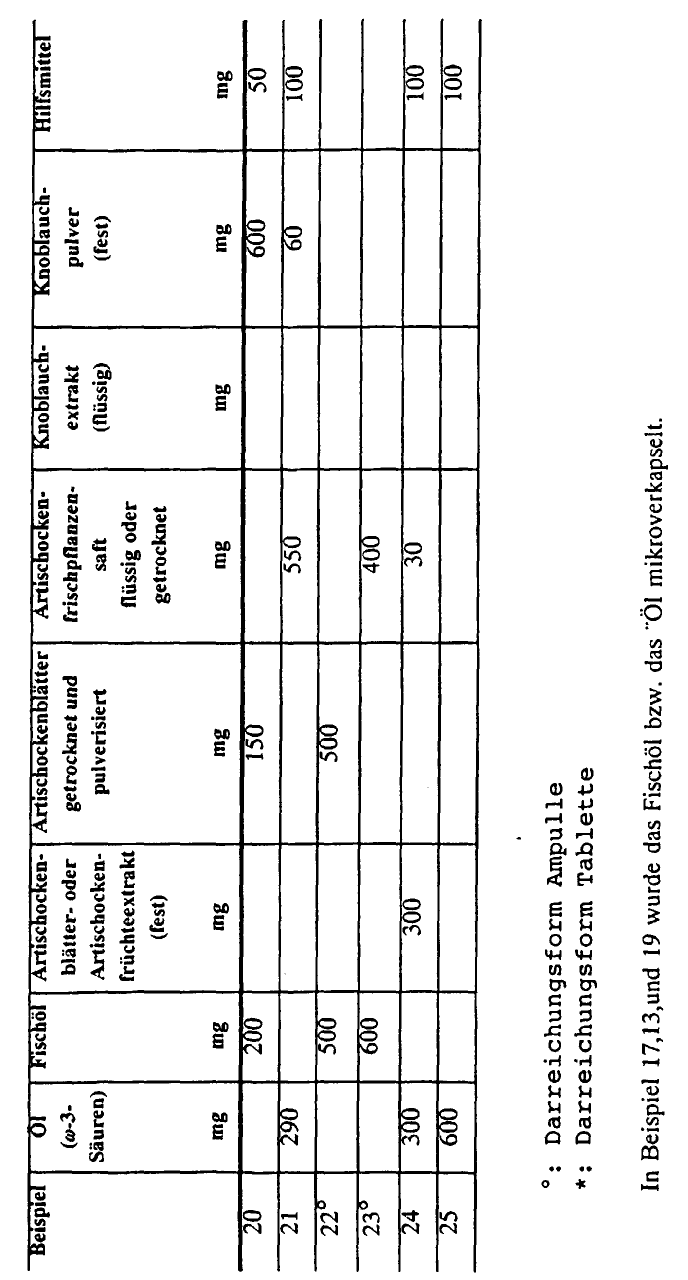

## Patentansprüche

1. Präparat bestehend aus mindestens einem Öl, welches mindestens eine mehrfach ungesättigte ω-3-Fettsäure in Form ihrer freien Säure, als Ester oder als Triglycerid enthält, und Bestandteilen aus der Artischockenpflanze.

2. Präparat bestehend aus mindestens einem Öl, welches mindestens eine mehrfach ungesättigte ω-3-Fettsäure in Form ihrer freien Säure, als Ester oder als Triglycerid enthält, und Bestandteilen aus der Artischockenpflanze zur Verwendung als Arzneimittel und/oder als Nahrungsergänzungsmittel.

3. Präparat nach Anspruch 1 oder 2, dadurch gekennzeichnet, dass das Öl die mehrfach ungesättigte ω-3-Fettsäure mindestens in einer Menge von 12 Gew.% bezogen auf das Gewicht des Öles enthält.

4. Präparat nach Anspruch 3, dadurch gekennzeichnet, dass das Öl Dokosahexaensäure in 5 bis 80% Gew.% und Eikosapentaensäure in einer Menge von 7 bis 60 Gew.% bezogen auf das Gewicht des Öls enthält.

5. Präparat enthaltend mindestens ein Seetieröl und Bestandteile der Artischockenpflanze (Cynara scolymus).

6. Präparat enthaltend mindestens ein Seetieröl und Bestandteile der Artischockenpflanze (Cynara scolymus) zur Verwendung als Arzneimittel und/oder Nahrungsergänzungsmittel.

7. Präparat nach Anspruch 5 oder 6, dadurch gekennzeichnet, dass das Seetieröl in einer Menge von 20 bis 80 Gew.% bezogen auf das Gesamtgewicht der jeweiligen Darreichungsform vorliegt.

8. Präparat nach einem der Ansprüche 5 bis 6, dadurch gekennzeichnet, dass das Seetieröl ein Fischöl ist, welches Dokosahexaensäure in 5 bis 80 Gew.% und Eikosapentaensäure in einer Menge von 7 bis 60 Gew.% bezogen auf das Gewicht des Fischöls enthält.

9. Präparat nach Anspruch 8, dadurch gekennzeichnet, dass das Fischöl aus dem Fischfleisch von Kaltwasserfischen, insbesondere von Makrelen, Hering, Anchovis und Thunfisch stammt.

10. Präparat nach einem der Ansprüche 1 bis 9, dadurch gekennzeichnet, dass die Bestandteile aus der Artischockenpflanze (Scynara scolymus) als getrocknete und zerriebene Artischockenblätter und/oder Artischockenfrüchte in einer Menge von 15 bis 60 Gew.% bezogen auf das Gesamtgewicht der Darreichungsform vorliegen.

11. Präparat nach einem der Ansprüche 1 bis 9, dadurch gekennzeichnet, dass die Bestandteile aus der Artischokkenpflanze (Scynara scolymus) als feste pulverförmige Extrakte aus den Artischockenblättern und/oder Artischockenfrüchten in einer Menge von 15 bis 60 Gew.% oder als Extrakte in Form hydrophiler oder hydrophober Lösungen oder Dispersionen in einer Menge von 15 bis 80% Gewicht % bezogen auf das Gesamtgewicht der Darreichungsform vorliegen.

12. Präparat nach einem der Ansprüche 1 bis 9, dadurch gekennzeichnet, dass die Bestandteile der Artischockenpflanze als flüssiger und/oder getrockneter Artischockenblätter- oder Artischockenfrüchte-Frischpflanzensaft in einer Menge von 30 bis 60 Gew.% bezogen auf das Gesamtgewicht der jeweiligen Darreichungsform des Präparates vorliegen.

13. Präparat nach einem der vorangegangenen Ansprüche, dadurch gekennzeichnet, dass das Präparat zusätzlich Bestandteile aus der Knoblauchpflanze (Allium sativum) in fester, pulverförmiger und/oder flüssiger Form in einer Menge von 5 bis 60 Gew.% bezogen auf das Gesamtgewicht der jeweiligen Darreichungsform des Präparates enthält.

14. Verwendung eines Präparates nach einem der vorangegangenen Ansprüche für die Herstellung eines Medikamentes zur Behandlung von Hyperlipidämie, insbesondere Hypercholesterinämie.
